# EUROPEAN PATENT SPECIFICATION

(11) **EP 2 011 859 B1**
(45) Date of publication and mention of the grant of the patent: **07.04.2010**
(21) Application number: 07120798.9
(22) Date of filing: 15.11.2007
(51) Int. Cl.: C12N 1/20, C12P 19/04

(54) **Pediococcus pentosaceus lactose-positive strain and a complex of fructan-containing exopolysaccharides synthesized by the strain**
Der Lactose-positive Stamm von Pediococcus pentosaceus und der davon synthesierte fruktanenenthaltende Komplex von Exopolysachariden
La souche positive de lactose Pediococcus pentosaceus et son complexe synthetise d'exopolysaccharides contenant des fructanes

(30) Priority: 05.07.2007 LV 070079
(43) Date of publication of application: 07.01.2009
(73) Proprietor: Latvijas Universitate, Riga 1098 (LV); Semjonovs, Pavels, Riga 1024 (LV); Zikmanis, Peteris, Riga 1012 (LV)
(72) Inventor: Semjonovs, Pavels, Riga 1024 (LV); Zikmanis, Peteris, Riga 1012 (LV)
(74) Representative: Fortuna, Aleksandra

(56) References cited:
- DI CAGNO RAFFAELLA ET AL: "Glucan and fructan production by sourdough Weissella cibaria and Lactobacillus plantarum" JOURNAL OF AGRICULTURAL AND FOOD CHEMISTRY, vol. 54, no. 26, December 2006 (2006-12), pages 9873-9881, XP002497308 ISSN: 0021-8561
- SMITINONT T ET AL: "Exopolysaccharide-producing lactic acid bacteria strains from traditional thai fermented foods: Isolation, identification and exopolysaccharide characterization" INTERNATIONAL JOURNAL OF FOOD MICROBIOLOGY, vol. 51, no. 2-3, 15 October 1999 (1999-10-15), pages 105-111, XP002497309 ISSN: 0168-1605
- VAN GEEL-SCHUTTEN G H ET AL: "Screening and characterization of Lactobacillus strains producing large amounts of exopolysaccharides" APPLIED MICROBIOLOGY AND BIOTECHNOLOGY, SPRINGER VERLAG, BERLIN, DE, vol. 50, no. 6, 1 December 1998 (1998-12-01), pages 697-703, XP002233876 ISSN: 0175-7598
- DATABASE EMBL [Online] 6 August 2007 (2007-08-06), "Pediococcus pentosaceus strain P 773 16S ribosomal RNA gene, partial sequence." XP002497311 retrieved from EBI accession no. EMBL:EU032327 Database accession no. EU032327
- SEMJONOVS PAVELS ET AL: "Evaluation of novel lactose-positive and exopolysaccharide-producing strain of Pediococcus pentosaceus for fermented foods" EUROPEAN FOOD RESEARCH AND TECHNOLOGY, vol. 227, no. 3, July 2008 (2008-07), pages 851-856, XP002497310 ISSN: 1438-2377

## Description

### Technical Field

Invention relates to a food biotechnology, in particular to production of synbiotic fermented functional food using the exopolysaccharides synthesizing *Pediococcus pentosaceus* strain, which expresses bifidogenic properties, improves functional properties of food products by means of fermentation carried out by lactose-positive strain of *Pediococcus pentosaceus* synthesizing exopolysaccharides or enriching the end product with exopolysaccharides (EPS) synthesized by the strain.

### Background Art

Lactic acid bacteria are widely used for production of fermented foods and improvement of functional properties of food products due to their technological, functional and therapeutic properties.

*Pediococcus pentosaceus* belongs to a group of homofermentative lactic acid bacteria. They are recognized as harmless to human health (GRAS - generally recognized as safe) and as safe for the use in food production. Strains of the species are found in natural microflora of spontaneously fermented food products such as dairy products, fermented meat and fish products, pickled vegetables, silage, and bread leavens. The lactic acid bacteria have a crucial role in forming taste and flavor of these products. *Pediococcus pentosaceus* is one of the most frequently isolated microorganisms from traditional bread leaven as well as other traditionally fermented products (Simpson, W.J. Taguchi, H. The genus Pediococcus, with notes on the genera Tetratogenococcus and Aerococcus. (1995) pp. 125-172. In: B.J.B. Wood and W.H. Holzapfel (eds). The genera of Lactic acid Bacteria. Chapman & Hall, London). Probiotic potential of *Pediococcus pentosaceus* strains had been estimated in human and animals. They had shown antibacterial activity against human intestinal pathogenic bacteria (Brink, M., Fraser, T. et al. A combined use of probiotics and prebiotics in a soymilk-based food supplement aimed at improving the gastrointestinal flora of children with HIV AIDS. Electron. J Environ Agric Food Chem. (2003) 2,4; 504-509.); they are used together with other bacteria in synbiotic preparations to maintain the gastrointestinal balance in humans (Bengmark, S., Gil, A. Bioecological and nutritional control of disease: prebiotics, probiotics and synbiotics. Nutr. Hosp. (2006) 21, 2; 72-84.). Addition of *Pediococcus pentosaceus* strains to porcine fodder significantly decreases amount of *Enterobacteriaceae* in the digestive tract, therefore they may be considered as potential probiotic supplement to porcine fodder (Gardiner, G.E., Casey, P.G. et al. Relative ability of orally administered Lactobacillus murinus to predominate and persist in the porcine gastrointestinal tract. Appl Environ Microbiol. (2004), 70 (4); 1895-1906.). Technological cultures containing *Pediococcus pentosaceus* are used for fermentation of sauerkrauts, cucumbers, beans, soya-based milk and silage. Use of these technological cultures for cabbage pickling decreases the biogenic amine level in them (Spicka, J., Kalac, P., et al. Application of lactic acid bacteria starter cultures for decreasing the biogenic amine levels in sauerkraut. Eur Food Res Technol. (2002) 215, 6, 509-514). Starters containing *Pediococcus pentosaceus* are widely used for production of fermented sausages since, due their technological properties, they act against the development of undesirable and pathogenic microflora (*Listeria, Pseudomonas, Staphylococcus, Enterobacteriaceae, Salmonella, Yersinia*), most probably, by means of synthesized bacteriocins (Mattila-Sandholm, T., Haikara A., Skytta, E. The effect of Pediococcus damnosus and Pediococcus pentosaceus on the growth of pathogens in minced meat. Int J Food Microbiol. (1991) 13,1; 87-94.) as well as are capable to reduce the level of residual nitrites. Strains of *Pediococcus pentosaceus* are among commercially available starter cultures employed for production of fermented meat products.

EPS of lactic acid bacteria and strains producing them are used to improve rheologic, organoleptic properties and texture of fermented products. For all that, EPS of lactic acid bacteria exhibit prebiotic, anticancer and immunity- stimulating activity as well as they possess ability to decrease cholesterol level in blood (Welman, A.D., Maddox I.S. Exopolysaccharides from lactic acid bacteria: perspectives and challenges. Trends in Biotechnology. (2003) 21, 8; 269-343.).

Currently the use of genetically modified organisms for improvement of technological and functional properties of food is limited. A wider use of *Pediococcus pentosaceus* strains in food biotechnology is substantially restricted because technologically important strains of this genus are typically unable to assimilate lactose. Due to this reason the lactose-negative strains of *Pediococcus pentosaceus* are not suitable for dairy starters (Caldwell, S.L., McMahon, D.J., et al. Development and Characterization of Lactose-Positive Pediococcus species for Milk Fermentation. Appl Environ Microbiol. (1996) 62, 3; 936-941.).
Currently strains of *Pediococcus pentosaceus* are used for the manufacturing of products where the inability to consume lactose is not crucial, e.g., lactose is not the principal substrate in the fermentation processes of meat products or sauerkraut. The use of genetically modified lactose positive strains is reported to substitute strains of *Streptococcus termophilus* in order to insure the phage-resistance of starters being essential in cheese manufacturing. Although there are no data on their commercial usage probably due to instability of recombinants and technological reasons (Caldwell, S.L., McMahon, D.J et al. Development and Characterization of Lactose-Positive Pediococcus species for Milk Fermentation. Appl Environ Microbiol. (1996) 62, 3; 936-941.).

VAN GEEL-SCHUTTEN G H ET AL: "Screening and characterization of Lactobacillus strains producing large amounts of exopolysaccharides" APPLIED MICROBIOLOGY AND BIOTECHNOLOGY, SPRINGER VERLAG, BERLIN, DE, vol. 50, no. 6, 1998-12-01, pages 697-703 reports production of significant amounts of exopolysaccharides containing glucan and fructan polymers by *Lactobacillus* strain LB 121.

The publication DI CAGNO RAFFAELLA ET AL: "Glucan and fructan production by sourdough Weissella cibaria and Lactobacillus plantarum" JOURNAL OF AGRICULTURAL AND FOOD CHEMISTRY, vol. 54, no. 26, 2006-12, pages 9873-9881 describes a strain of *Weissella cibaria* which produces a complex of exopolysaccharides containing glucan and fructan polymers.

Currently there are no commercially available starters containing exopolysaccharides-producing strains of *Pediococcus pentosaceus.* The presence of EPS producing strains would be very desirable in bread and meat starters because they could significantly improve the quality of final products by conferring them prebiotic properties. Probiotic microorganisms (live microorganisms in food products as well as food supplements containing these organisms) positively affect a microbial balance in the digestive tract of humans and animals,thus improving health and wellbeing of the host organisms (Mason, P. Probiotics and Prebiotics. The Pharmaceutical Journal. (2001) 266, 7132; 118-121).

Fructans are proved to possess prebiotic and other properties positively affecting human health (Kaur, N., Gupta, A.K. Application of inulin and oligofructose in health and nutrition. J Biosci. (2002) 27, 7; 703-714), however there are no commercially available starters containing the fructan-producing strains. The presence of fructans in the EPS complex of *Pediococcus pentosaceus* P 773 could significantly improve functional properties of food products especially their prebiotic and probiotic properties, thus positively affecting human health.

### Disclosure of the invention

There has been isolated a new homofermentative lactose-positive strain of *Pediococcus pentosaceus -* lactic acid bacteria producing fructan-containing exopolysaccharides complex. The microbial strain had been deposited at the international depositary - Microbial Strain Collection of Latvia (MSCL) since the 29th of March 2007 (accession number P 773). The newly isolated *Pediococcus pentosaceus* strain P 773 assimilates lactose as the sole carbon source and grows well on various food-grade substrates - in milk, cereal, fruit and vegetable juices, soymilk and others. Due to these properties the strain appears as a suitable technological culture for all kinds of fermenting processes, both as the individual starter culture or in combination with other cultures. The further aspect of the invention is the method for synthesizing of fructan-containing exopolysaccharides complex with prebiotic properties by the use of the *Pediococcus pentosaceus* strain P 773.

### Description of figures

Fig. 1 shows a homology and phylogenetic tree of *Pediococcus pentosaceus* where the strain is identified by the 16S rDNA sequence comparisons.
Fig. 2 reflects biochemical characteristics of the *Pediococcus pentosaceus* strain indicated by the API 50CHL identification system.
Fig. 3 shows the gel-filtration profile of exopolysaccharides produced by the *Pediococcus pentosaceus* strain.

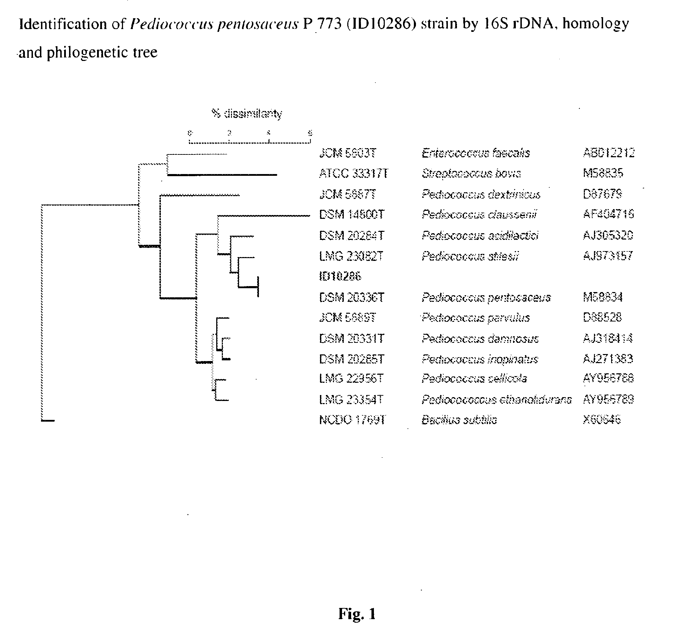

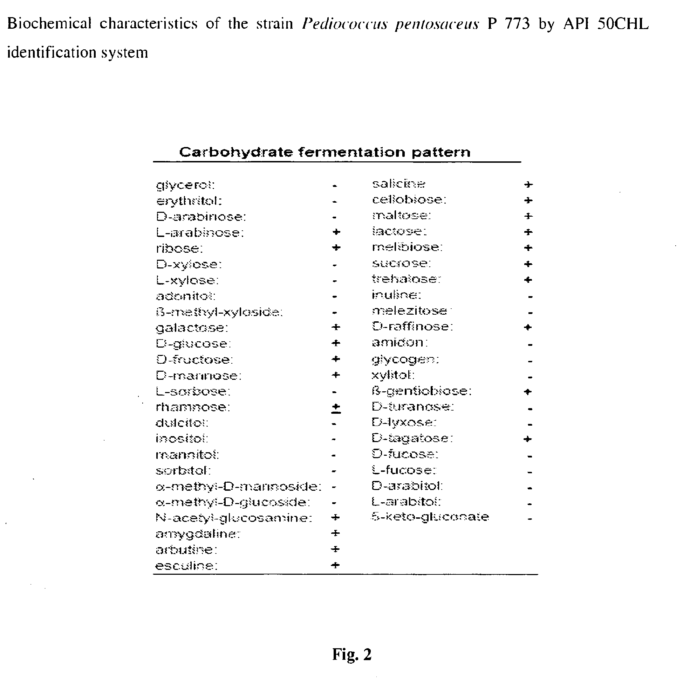

The lactose-positive strain of *Pediococcus pentosaceus* is isolated from spoiled beer and identified by the 16S rDNA sequence comparisons (Fig.1) and carbohydrate fermentation patern using the API 50CHL identification system (Fig.2) to the species level.

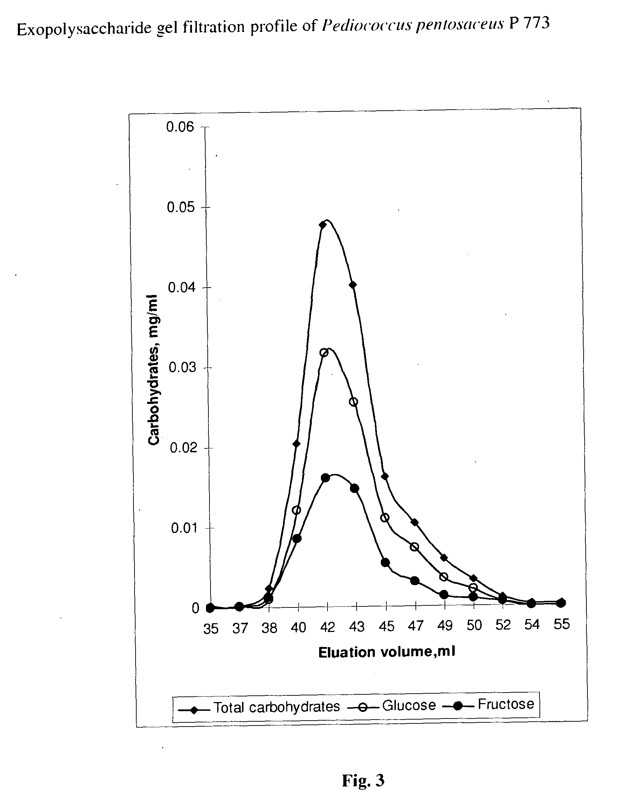

The strain produces a mixture of EPS represented by fructan and glucan (Fig.3) in the presence of sucrose or raffinose. The strain can consume lactose as a sole carbon source (Table 1).

**Table 1.**

| Influence of *Pediococcus pentosaceus* P 773 exopolysaccharydes (EPS) 20.0 g/L on the specific growth rate of probiotic dairy starter cultures in the modified MRS-lactose (45.0 g/L) medium | | |
|---|---|---|
| Cultures | Specific growth rate - µ, h⁻¹ | |
| | Lactose (control) | Lactose + EPS |
| *B. lactis* | 0.18±0.01 | 0.22±0.01 |
| *L. acidophilus* | 0.50±0.01 | 0.55±0.01 |
| *S. thermophilus* | 0.59±0.01 | 0.67±0.02 |

Exopolysaccharides synthesized by the strain promote the growth of lactic acid bacteria, including the growth of probiotic cultures (Table 2) either by EPS synthesized *in situ* during fermentation process or by addition of them to the product at any stage of production. The above mentioned EPS can serve for lactic acid bacteria an bifidobacteria as a sole carbon source (Table 1). EPS synthesized by the strain can be added to the product during the production process as a functional and technological additive.

**Table 2.**

| Assimilation of *Pediococcus pentosaceus* P 773 exopolysaccharydes as a sole carbon source by common constituent cultures of probiotic dairy starters in the modified MRS medium (glucose replaced by 20.0 g/L EPS (48 h) | | | |
|---|---|---|---|
| Cultures | Biomass, g/L | Lactic acid, g/L | Consumption of carbohydrates, % of initial |
| *B. lactis* | 0.91±0.04 | 3.15±0.17 | 19.15±1.12 |
| *L. acidophilus* | 0.30±0.01 | 3.24±0.16 | 7.45±0.52 |
| *S. thermophilus* | 0.74±0.02 | 3.78±0.19 | 23.40±1.52 |

The strain grows well on different food substrates (Table 3) and is appropriate for production of synbiotic functional products. Overall properties of the strain comprise characteristics which are especially perspective in food manufacturing.

**Table 3.**

| Characteristics of *Pediococcus pentosaceus* P 773 during 48 h cultivation on different carbon sources (100 g/L) in the modified MRS media | | | | |
|---|---|---|---|---|
| | Carbon source in the medium | | | |
| | Sucrose | Rafinose | Lactose | Glucose |
| Biomass, g/L | 6.1±0.2 | 3.2±0.1 | 2.4±0.1 | 2.5±0.1 |
| EPS synthesis, g/L | 3.9±0.15 | 1.2±0:4 | -* | -* |
| Lactic acid, g/L | 17.5±0.7 | 24.7±0.9 | 26.6±0.8 | 26.3±0.9 |
| Carbohydrate consumption, g/L | 63.0±3.1 | 48.1±2.2 | 44.0±2.0 | 50.0±2.8 |
| Biomass yield-Yx/s, g/g | 0.10 | 0.07 | 0.05 | 0.06 |
| Acid yield-Yx/s, g/g | 0.28 | 0.51 | 0.53 | 0.60 |

| | | | | |
|---|---|---|---|---|
| * EPS are not synthesized | | | | |

EPS synthesized by the strain have been assessed and qualified as non-toxic by the Diagnostic Centre of the Latvian Food and Veterinary Authority. Introduction of the strain into manufacturing practices would allow to broaden a choice of functional food products, to reduce production costs of synbiotic food products, to improve a quality and safety of traditional products (e.g. due to the synthesis of bacteriocins) and to diversify organoleptic properties of foods (Table 4).

**Table 4.**

| Fermentation (42 h) of various food substrates by *Pediococcus pentosaceus* P 773 | | |
|---|---|---|
| Food substrate fermented | Cell count*, log_{cfu}/mL | Lactic acid, g/L |
| Cow's milk | 8.85±0.035 | 8.82±0.45 |
| Goat's milk | 8.84±0.037 | 12.96±0.55 |
| Soymilk | 7.08±0.030 | 5.58±0.22 |
| Carrot juice | 9.11±0.042 | 12.24±0.58 |
| Apple juice | 7.28±0.030 | 10.08±0.52 |
| Oat substrate | 8.06±0.039 | 2.70±0.11 |
| Barley substrate | 7.92±0.035 | 3.06±0.12 |
| Pease substrate | 9.01±0.046 | 4.32±0.25 |
| Buckwheat substrate | 7.93±0.040 | 3.06±0.13 |
| Rye substrate | 7.49±0.038 | 3.06±0.12 |

| | | |
|---|---|---|
| *Initial cell count 6.22 log_{cfu}/mL | | |

For synthesis of the complex of exopolysaccharides with prebiotic properties *Pediococcus pentosaceus* P 773 strain is cultivated in the growth medium at a range of temperatures 15° - 42° C, preferably 30° - 37° C, more preferably 37° C, for 12 - 72 hours, preferably 36 - 60 hours. The growth medium contains sucrose or raffinose at the range of concentration 0.1 - 120 g/L; maximal outcome of polymers is achieved if sucrose or raffinose concentration is at the range 100 - 120 g/L.

The cultivation method of the strain *Pediococcus pentosaceus* P 773 and the method of synthesis of the fructan-containing exopolysaccharides complex with prebiotic properties using the strain *Pediococcus pentosaceus* P 773 are explained by examples.

### Example 1

*Pediococcus pentosaceus* strain P 773 is cultivated in the MRS liquid medium (DSMZ, Medium 11) containing (g/L): casein peptone (tryptic digest), 10.00; meat extract , 10.00; yeast extract, 5.00; glucose, 20.00; Tween 80, 1; K₂HPO₄, 2; Sodium acetate, 5.00; (NH₄)₂citrate, 2.00; MgSO₄ x 7 H₂O, 0.20; MnSO₄ x H₂O, 0.05; distilled water, 1000 ml. After the sterilization of the medium, pH is adjusted by HCl or NaOH to 6.2 - 6.5. Viability of cells, contamination tests, morphological and cell-count assessment are performed using agarized (12.0 g/L) basic medium (e.g., MRS or any other suitable for the growth of the strain). Optimal growth temperature is 37° C, however the strain grows also in lower or higher temperature. The strain is grown in anaerobic (BBL Gas Pak 150TM System, USA) or semi-aerobic conditions. Glucose, the principal source of carbon in the MRS medium, can be substituted by other appropriate carbon source, e.g., lactose, sucrose, rafinose at the concentration range of 10 - 120 g/L.

### Example 2

Synthesis of EPS by *Pediococcus pentosaceus* P 773 is performed in 21 flask containing the modified MRS medium with 120 g/L sucrose or 120 g/L rafinose, at 37°C under anaerobic conditions (BBL Gas Pak 150TM System, USA). Two percent of overnight culture (7.8 - 8.0 10g_{cfu}/mL) grown in the modified MRS medium is used as an inoculum. After 72 h semianaerobic cultivation in the MRS-sucrose medium, when a concentration of cells is close to 1g/L, cells are removed by centrifugation (5000 r/min, 30 min) and three volumes of chilled 96 % ethanol are added to the supernatant. After overnight incubation at 4° C, the precipitate is collected by centrifugation (5000 r/min, 30 min). The pellet is dissolved in deionized water and twice reprecipitated by ethanol (1 : 3 v/v). In order to obtain EPS of higher purity precipitate is diluted again and dialysis of the solution can be performed.

### Example 3

The inoculum of *Pediococcus pentosaceus* P 773 is added to pasteurized cow's or goat's milk in amount of 2% (overnight culture in MRS medium). An incubation is carried out for 48 hours at temperature 37° C. At the end of cultivation fermented milk contains 8.8 log cfu/mL of *Pediococcus pentosaceus* P 773 cells and 8-13 g/L lactic acid produced during fermentation (see Table 4). A development of *Pediococcus pentosaceus* P 773 in milk is enhanced by an addition of yeast extract, casein hydrolysate, glucose or sucrose.

### Example 4

100 ml of distilled water is added to oat, rye, barley, buckwheat or peas flour (5g), thoroughly stirred and let to swell for 6 hours at the temperature 4° C. Substrates are sterilized at 121 °C for 20 minutes. Inoculum of *Pediococcus pentosaceus* P 773 is added to cereal or pulses substrates in amount of 2% (overnight culture in the MRS medium). Incubation is carried out for 48 hours at temperature 37° C. At the end of fermentation cereal or pulses substrates contain 7.0-9.0 log cfu/mL of live *Pediococcus pentosaceus* P 773 cells and lactic acid (see Table 4). A development of *Pediococcus pentosaceus* P 773 cells on cereal or pulses substrates is enhanced by treating them with α-amylase, yeast extract, casein hydrolysate, glucose, sucrose or by addition of milk.

### Example 5

Freshly squeezed carrot or apple juice is pasteurized, *Pediococcus pentosaceus* P 773 inoculum is added (overnight culture in MRS medium) in amount of 2%. Incubation is carried out for 48 hours at temperature 37° C. At the end of fermentation carrot or apple juices contain 7.2 to 9.1 log cfu/mL of *Pediococcus pentosaceus* P 773 cells as well as 10-12 g/L of lactic acid (see Table 4).

### Example 6

MRS medium is modified by substituting of 20.0 g/L glucose to 45.0 g/L lactose and adding 20.0 g/L preparation of EPS of *Pediococcus pentosaceus* P 773. The medium is sterilized at 0.5 atm. for 15 minutes. Inocula of *Bifidobacterium lactis, Lactobacillus acidophilus* or *Streptococcus thermophilus* in amount of 2% are introduced into medium (overnight culture in MRS medium). Incubation is carried out at 37° C under anaerobic conditions (BBL Gas Pak 150TM System, USA). The rate of culture biomass formation is monitored by a spectrophotometric method (Shimadzu UV-vis, Japan) at λ=550 nm. In the presence of EPS produced by *Pediococcus pentosaceus* P 773. an increase of specific growth rate (µ, h⁻¹) of *Bifidobacterium lactis, Lactobacillus acidophilus* or *Streptococcus thermophilus* cultures is determined and compared to the control culture containing lactose as a sole carbon source (see Table 1).

## Claims

1. The strain of *Pediococcus pentosaceus* deposited in Microbial Strain Collection of Latvia (MSCL) under the accession number P 773.

2. Products containing the strain Pediococcus pentosaceus P 773 of claim 1 and the complex of exopolysaccharides containing glucan and fructan polymers synthesized by the strain of claim 1.

3. The method of synthesis of the complex of exopolysaccharides containing glucan and fructan polymers with prebiotic properties, **characterized in that** the strain mentioned in claim 1 is cultivated at a range of temperatures 15° - 42° C, preferably 30° - 37° C, more preferably 37° C, for 12 - 72 hours, preferably 36 - 60 hours, where the growth medium contains sucrose or raffinose at the range of concentration 0.1 - 120 g/L.

## Patentansprüche

1. Der in der Mikroorganismensammlung von Lettland "MSCL" (Microbial Strain Collection of Latvia) unter der Zugangsnummer P 773 hinterlegte Stamm von *Pediococcus pentosaceus.*

2. Produkte, die den Stamm Pediococcus pentosaceus P 773 aus Anspruch 1 und den aus dem Stamm von Anspruch 1 synthetisierten, Glucan- und Fructan-Polymere enthaltenden Komplex von Exopolysacchariden enthalten.

3. Verfahren zur Synthese des Glucan- und Fructan-Polymere enthaltenden Komplexes von Exopolysacchariden mit prebiotischen Eigenschaften, **dadurch gekennzeichnet, dass** der in Anspruch 1 erwähnte Stamm 12 - 72 Stunden, vorzugsweise 36 - 60 Stunden lang bei einer Temperatur im Bereich von 15° bis 42° C, vorzugsweise zwischen 30° und 37° C, noch bevorzugter bei 37° C, kultiviert wird, wobei das Nährmedium Sukrose oder Raffinose in der Konzentration von 0,1 bis 120 g pro Liter enthält.

## Revendications

1. Souche de *Pediococcus pentosaceus* déposée dans la Collection de Souches Microbiennes de la Lettonie (CSML) sous le numéro d'accès P 773.

2. Produits contenant la souche Pediococcus pentosaceus P 773 de la revendication 1 et le complexe d'exopolysaccharides contenant des polymères de glucane et de fructane synthétisés par la souche de la revendication 1.

3. Méthode de synthèse du complexe d'exopolysaccharides contenant des polymères de glucane et de fructane présentant des propriétés prébiotiques, **caractérisée en ce que** la souche mentionnée dans la revendication 1 est cultivée à une plage de températures de 15° à 42°C, de préférence de 30° à 37°C, de manière très préférée à 37°C, pendant 12 à 72 heures, de préférence 36 à 60 heures, le milieu de culture contenant du saccharose ou du raffinose à une concentration de 0,1 à 120 g/L.


### Sequence Listing

<110> LATVIJAS UNIVERSITATE; SEMJONOVS, Pavels; ZIKMANIS, Peteris
<120> *Pediococcus pentosaceus* lactose-positive strain and a complex of fructan-containing exopolysaccharides synthesized by the strain
<130> LAP 2/1221
<140>
   <141>
<150> LV P-07-79
   <151> 2007-07-05
<160> 1
<170> MS Word
<210> 1
   <211> 807
   <212> DNA
   <213> *Pediococcus pentosaceus*
<220>
   <221>
   <222>
<300>
   <301>
   <302>
   <303>
   <304>
   <305>
   <306>
   <307>
   <308> EU032327
   <309> August 6, 2007
<400> 1

gatgaacgct ggcggcgtgc ctaatacatg caagtcgaac gaacttccgt taattgatta 60
tgacgtactt gtactgattg agattttaac acgaagtgag tggcgaacgg gtgagtaaca 120
cgtgggtaac ctgcccagaa gtaggggata acacctggaa acagatgcta ataccgtata 180

acagagaaaa ccgcatggtt ttcttttaaa agatggctct gctatcactt ytggatggac 240
ccgcggcgta ttagctagtt ggtgaggtaa aggctcacca aggcagtgat acgtagccga 300
cctgagaggg taatcggcca cattgggact gagacacggc ccagactcct acgggaggca 360
gcagtaggga atcttccaca atggacgcaa gtctgatgga gcaacgccgc gtgagtgaag 420
aagggtttcg gctcgtaaag ctctgttgtt aaagaagaac gtgggtaaga gtaactgttt 480
acccagtgac ggtatttaac cagaaagcca cggctaacta cgtgccagca gccgcggtaa 540
tccggattta ttgggcgtaa agcgagcgca ggcggtcttt taagtctaat gtgaaagcct 600
tcggctcaac cgaagaagtg cattggaaac tgggagactt gagtgcagaa gaggacagtg 660
gaactccatg tgtagcggtg aaatgcgtag atatatggaa gaacaccagt ggcgaaggcg 720
gctgtctggt ctgcaactga cgctgaggct cgaaagcatg ggtagcgaac aggattagat 780
accctgg 840